# EUROPEAN PATENT SPECIFICATION

(11) **EP 2 701 252 B1**
(45) Date of publication and mention of the grant of the patent: **01.01.2020**
(21) Application number: 12864678.3
(22) Date of filing: 25.06.2012
(51) Int. Cl.: F24F 1/0076, H01T 23/00, H01T 19/00, B03C 3/41, F24F 1/02, A61L 9/22, F24F 1/00, H05H 1/48, B03C 3/09, B03C 3/12, B03C 3/38, B03C 3/47

(54) **AIR CONDITIONER COMPRISING AN ELECTRIC DISCHARGE APPARATUS**
KLIMAANLAGE MIT EINER VORRICHTUNG FÜR ELEKTRISCHE ENTLADUNG
CLIMATISEUR AVEC SON DISPOSITIF DE DÉCHARGE

(43) Date of publication of application: 26.02.2014
(73) Proprietor: Mitsubishi Electric Corporation, Tokyo 100-8310 (JP)
(72) Inventor: FURUHASHI, Takuya, Tokyo 100-8310 (JP); MORIOKA, Reiji, Tokyo 100-8310 (JP); SAIKI, Ayumi, Tokyo 100-8310 (JP)
(74) Representative: Pfenning, Meinig & Partner mbB
(86) International application number: PCT/JP2012/066126
(87) International publication number: WO 2014/002158

(56) References cited:
- JP-A- H0 838 939
- JP-A- H03 146 148
- JP-A- H04 171 064
- JP-A- H05 161 857
- JP-A- 2005 134 648
- JP-A- 2009 230 997
- JP-U- S6 283 551

## Description

### Technical Field

The present invention relates to a discharge device.

### Background Art

An electric precipitator has been proposed including an ionized wire formed by swaging crimping terminals at opposite ends of a discharge member. The electric precipitator allows a loop portion of the crimping terminal to be easily hung on a spring (for example, see Patent Literature 1).

However, in the electric precipitator described in Patent Literature 1, the discharge member tends to be cut at the end of the crimping terminal.

In contrast to this, an air cleaner is proposed in which a metal pipe is crimped to loop portions formed by bending opposite ends of a discharge member. In this air cleaner, a crimp portion between the end of the discharge member and the metal pipe is covered with an electrically insulating resin pipe. This can prevent the discharge member from being cut at the end of the metal pipe (for example, see Patent Literature 2).

JP S62 83551 U discloses a discharge electrode which comprises a discharge member and a loop like terminal mounted to an end of the discharge member. The discharge electrode further has a tube made of a resin and covering a mounting portion between an end of the discharge member and the terminal.

### Citation List

### Patent Literature

Patent Literature 1: Japanese Patent Laid-Open No. 8-38939
Patent Literature 2: Japanese Patent Publication No. 6-38927

### Summary of Invention

### Technical Problem

However, in the air cleaner described in Patent Literature 2, it is difficult to form uniform loops at the opposite ends of the discharge member. Thus, a large loop must be formed to be hung on a spring. Meanwhile, forming a large loop increases the size of the device. Further, there is a need to create a spatial distance and a creepage distance between the loop and a counter electrode. This further increases the size of the device.

The present invention is made to solve the above described problems, and aims to provide an air conditioner comprising a discharge device that can prevent cutting of a discharge member, and allows both a reduction in thickness and an improvement in assemblability.

### Means for Solving the Problems

The above problems are solved by the subject-matter according to the independent claim. A discharge device includes a discharge electrode and a counter electrode facing each other, wherein the discharge electrode includes a discharge member facing the counter electrode, a loop-like terminal mounted to an end of the discharge member in a longitudinal direction, and a tube having an electrically insulating property and covering a mounting portion between an end of the discharge member and the terminal.

### Advantageous Effects of Invention

The present invention can prevent cutting of a discharge member, and allows both a reduction in thickness and an improvement in assemblability.

### Brief Description of the Drawings

Figure 1 is an exploded perspective view of a discharge device in Example 1.
Figure 2 is a perspective view of an inside of the discharge device in Example 1.
Figure 3 is a plan view of the end of the discharge electrode of the discharge device in Example 1.
Figure 4 is a schematic view showing a positional relationship between the end of the discharge electrode and the counter electrode in the discharge device of Example 1.
Figure 5 is a vertical sectional view of an indoor unit of an air conditioner including a discharge device according to Embodiment 1 of the present invention.

### Description of Embodiments

Embodiments of the present invention will be described with reference to the accompanying drawings. The same reference numerals denote the same or corresponding components throughout the drawings, and overlapping descriptions will be simplified or omitted.

### Example 1

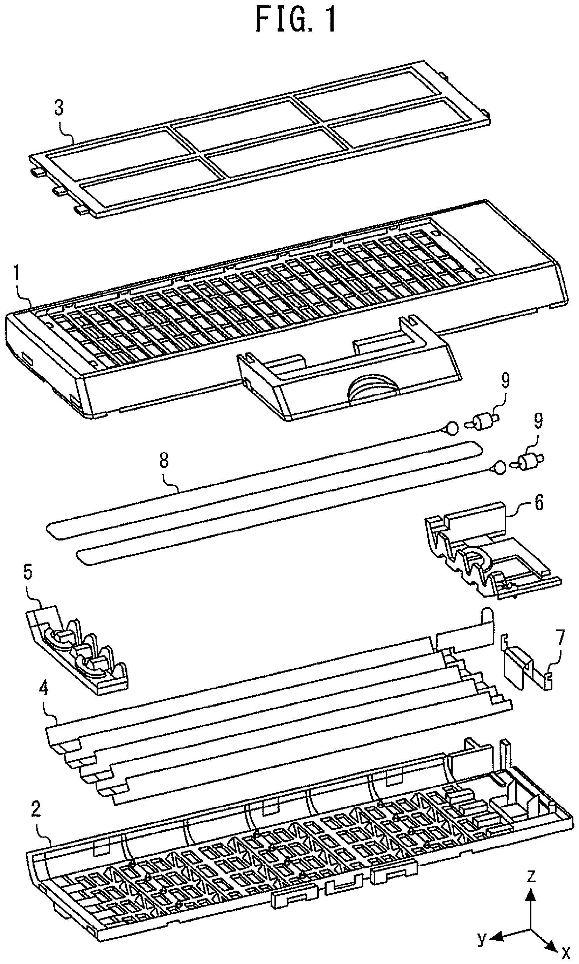

Figure 1 is an exploded perspective view of a discharge device in Example 1. Example 1 is helpful for understanding certain aspects of the present invention. A positive side of a Z axis in Figure 1 is a windward side. A negative side of the Z axis in Figure 1 is a leeward side.

As shown in Figure 1, an outline of the discharge device is formed of an upper frame 1 and a lower frame 2.

The upper frame 1 is placed on the most windward side of the discharge device. For example, the upper frame 1 is entirely made of resin. The upper frame 1 includes a lattice. The lattice is formed to prevent a human's finger from entering the discharge device. Each hole partitioned by the lattice is an opening portion. The opening portion is formed so as to take air outside the discharge device into the discharge device. Specifically, the opening portion is formed in an upper surface (a surface parallel to an X-Y plane in Figure 1) of the upper frame 1 over a predetermined width (a distance in an X axis direction in Figure 1) and a predetermined depth (a distance in a Y axis direction in Figure 1). A handle is provided in the upper frame 1. The handle is formed so as to be gripped by a human when holding the discharge device by hand.

The lower frame 2 is placed on the most leeward side of the discharge device. For example, the lower frame 2 is entirely made of resin. The lower frame 2 includes a lattice. The lattice is formed to prevent a human's finger from entering the discharge device. Each hole partitioned by the lattice is an opening portion. The opening portion is formed so as to discharge air taken into the discharge device to the outside of the discharge device. Specifically, the opening portion is formed in a lower surface (a surface parallel to the X-Y plane in Figure 1) of the lower frame 2 over a predetermined width (a distance in the X axis direction in Figure 1) and a predetermined depth (a distance in the Y axis direction in Figure 1).

The surface (opening surface) of the lower frame 2 having the opening portion is placed in parallel with the surface (opening surface) of the upper frame 1 having the opening portion. Without any parts in the discharge device, each opening portion in the lower frame 2 faces each opening portion in the upper frame 1 at a predetermined distance in a height direction (a Z axis direction in Figure 1).

A prefilter 3 is fitted in a front surface of the upper frame 1. The prefilter 3 is formed of mesh finer than the lattice of the opening surface of the upper frame 1.

The discharge device includes therein a counter electrode 4, a first electrode support unit 5, a second electrode support unit 6, a power feeding unit 7, a discharge electrode 8, and a spring 9.

The counter electrode 4 is placed to face the lower frame 2. For example, the first electrode support unit 5 is entirely made of resin. The first electrode support unit 5 secures the end of one side on the counter electrode 4. For example, the second electrode support unit 6 is entirely made of resin. The second electrode support unit 6 secures the other end of the counter electrode 4.

The power feeding unit 7 is made of metal. The power feeding unit 7 is mounted to the other end of the lower frame 2 so as not to interfere with the second electrode support unit 6. The discharge electrode 8 is held in a proper position, with an intermediate portion being folded back about twice to four times, by a member of the first electrode support unit 5 and a member of the second electrode support unit 6. The spring 9 is made of metal. The spring 9 is coupled to the power feeding unit 7 and an end of the discharge electrode 8. The spring 9 applies predetermined tension to the discharge electrode 8 in a longitudinal direction.

In the discharge device, a voltage of 4 to 7 kV is applied to the discharge electrode 8 with the counter electrode 4 being grounded. This forms an electric field between the discharge electrode 8 and the counter electrode 4. The electric field generates corona discharge.

At this time, a blast fan (not shown) placed outside the discharge device is driven, and then air outside the discharge device passes through the prefilter 3. The air is taken from the opening portion in the upper frame 1 into the discharge device. At this time, bacteria, mold, viruses, or the like suspended in the air are also taken from the opening portion in the upper frame 1 into the discharge device.

The bacteria, mold, viruses, or the like pass through between the discharge electrode 8 and the counter electrode 4. At this time, an electric field and a discharge are applied to the bacteria, mold, viruses, or the like. The electric field and the discharge destroy the bacteria, mold, viruses, or the like. This kills the bacteria, mold, viruses, or the like. The killed bacteria, mold, viruses, or the like are discharged with air from the opening portion in the lower frame 2 to the outside of the discharge device. This removes and inactivates living bacteria, mold, viruses, or the like suspended in a room.

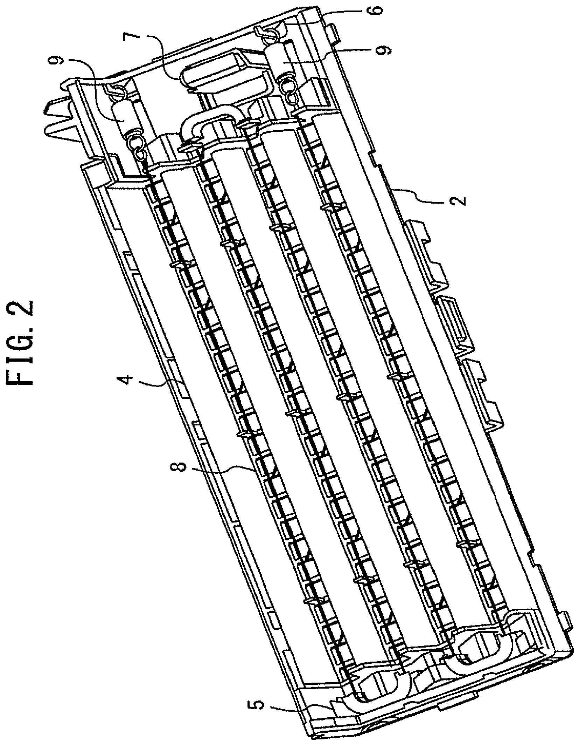

Next, with reference to Figure 2, the counter electrode 4 will be described.

Figure 2 is a perspective view of an inside of the discharge device in Example 1. In Figure 2, the upper frame 1 and the prefilter 3 are removed.

The counter electrode 4 is fabricated by cutting and bending a plate-like metal member. For example, the counter electrode 4 is preferably made of tungsten, copper, nickel, stainless, zinc, iron, molybdenum, or the like. The counter electrode 4 may be made of alloys mainly consisting of the above described metal, or the above described metal plated with noble metal such as silver, gold, or platinum.

The counter electrode 4 is tilted by a predetermined angle with respect to the opening surface of the upper frame 1 and the opening surface of the lower frame 2. In this example, the counter electrode 4 has an angle of 45° to 60° with respect to the opening surface of the upper frame 1 and the opening surface of the lower frame 2.

The metal counter electrode 4 is more precisely formed than a counter electrode 4 by resin molding. Changes in tilt with time of the metal counter electrode 4 are more significantly prevented than changes in tilt with time of the counter electrode 4 by resin molding.

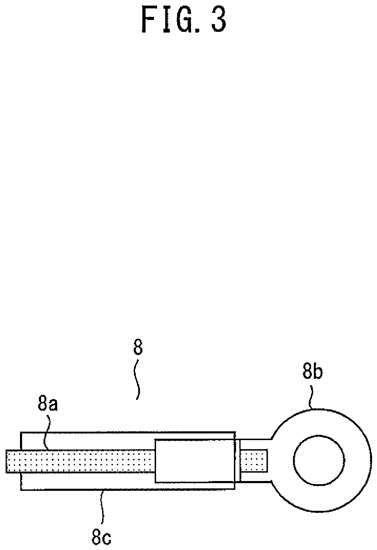

Next, with reference to Figure 3, the discharge electrode 8 will be described.

Figure 3 is a plan view of the end of the discharge electrode of the discharge device in Example 1.

As shown in Figure 3, the discharge electrode 8 includes a discharge member 8a, a terminal 8b, and a tube 8c.

The discharge member 8a is formed of a long plate-like or linear metal member. For example, the discharge member 8a is preferably made of tungsten, copper, nickel, stainless, zinc, iron, molybdenum, or the like. The discharge member 8a may be made of alloys mainly consisting of the above described metal, or the above described metal plated with noble metal such as silver, gold, or platinum, or having a carbon (graphite) layer or an oxide film.

The discharge member 8a as a plate-like member has a rectangular section surrounded by short and long sides. For example, in the section of the discharge member 8a, the short side has a length of 0.01 to 0.1 mm. The long side has a length of 0.1 to 1.0 mm.

The terminal 8b is looped so as to be easily mounted to the spring 9. The terminal 8b is mounted to each end of the discharge member 8a by crimping or welding. For example, the terminal 8b is made of stainless, nickel, tin, aluminum, brass, copper, or the like. The terminal 8b may be made of alloys mainly consisting of the above described metal, or the above described metal plated with noble metal such as silver, gold, or platinum, or plated with stainless, nickel, tin, aluminum, brass, copper, or the like.

The tube 8c has an electrically insulating property. The tube 8c covers a mounting portion between the discharge member 8a and the terminal 8b. The tube 8c covers the discharge member 8a by a predetermined length from the mounting portion of the terminal 8b toward a center of the discharge member 8a in the longitudinal direction.

The tube 8c is made of resin that does not generate ions by heat and has low hygroscopicity. For example, the tube 8c is a heat shrinkable tube having flexibility, a tube having an inner surface to which an adhesive is applied, a heat shrinkable tube having an inner surface to which an adhesive is applied, or the like. The tube 8c desirably has such flexibility as to be bent to about 90° by hand.

When the tube 8c is a heat shrinkable tube, the tube 8c is heated through the discharge member 8a and the terminal 8b. The heating shrinks the tube 8c. Thus, the tube 8c is secured to the discharge member 8a and the terminal 8b without any space therebetween.

When the tube 8c is a tube having an inner surface to which an adhesive is applied, the tube 8c is reliably secured to the discharge member 8a and the terminal 8b without any space therebetween.

Silicone or the like may be poured into the space between the tube 8c, the discharge member 8a, and the terminal 8b. In this case, the silicone fills the space.

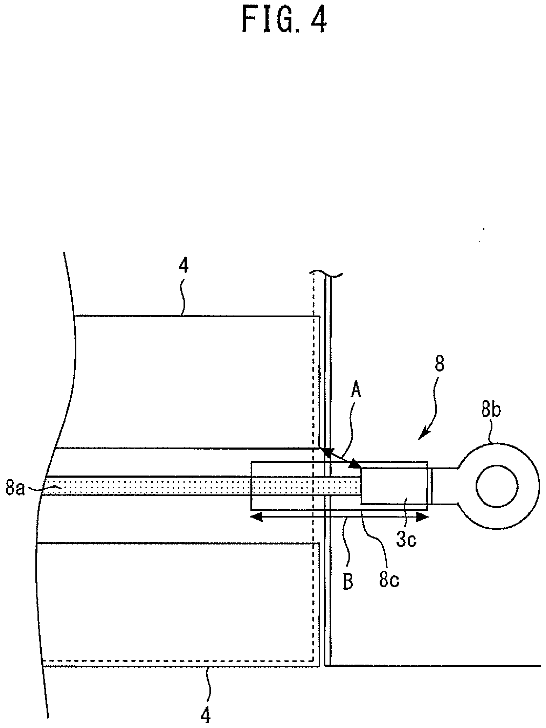

Next, with reference to Figure 4, a positional relationship between the counter electrode 4 and the discharge electrode 8 or the like will be described in detail.

Figure 4 is a schematic view showing a positional relationship between the end of the discharge electrode and the counter electrode in the discharge device of Example 1.

In Figure 4, reference character A denotes a shortest distance between the terminal 8b and the end of the counter electrode 4. Reference character B denotes a length of the tube 8c. A left upper portion indicated by a broken line is a space (air trunk) through which air taken into the discharge device passes.

As shown in Figure 4, an end of the terminal 8b on a side of the discharge member 8a is placed outside the counter electrode 4 when seen in a facing direction of the discharge member 8a and the counter electrode 4. In this state, the length B is longer than the shortest distance A. Thus, the tube 8c covers the discharge member 8a up to the space indicated by the broken line, through which air taken into the discharge device passes. Specifically, the end of the tube 8c on the side of the discharge member 8a is placed closer to the space through which air passes than the end of the counter electrode 4.

According to Example 1 described above, the mounting portion between the end of the discharge member 8a and the terminal 8b is covered with the tube 8c. Specifically, the tube 8c covers the discharge member 8a by a predetermined length from the mounting portion between the end of the discharge member 8a and the terminal 8b toward the center of the discharge member 8a in the longitudinal direction. This increases strength of the discharge member 8a at the end of the terminal 8b. This can prevent cutting of the discharge member 8a. Also, the tube 8c can be fitted in a slit in the second electrode support unit 6. This improves assemblability of the discharge electrode 8. Thus, yields in assembling are improved. At the same time, a discharge portion can be located to the maximum in the space through which air passes. This can reduce an area of a portion supporting the discharge electrode 8. Thus, a thickness of the discharge device can be reduced to form a stable uniform electric field.

Also, the end of the tube 8c is secured to the mounting portion between the end of the discharge member 8a and the terminal 8b without any space therebetween by heat shrinkage or bonding. This can prevent air and water from entering a fitting portion between the discharge member 8a and the terminal 8b, prevent development of corrosion of the discharge member 8a and the terminal 8b, and reliably prevent a problem such as disconnection. Also, even in the case of loose joining between the discharge member 8a and the terminal 8b, the joining can be held by bonding, thereby allowing energization and maintaining performance over a long period.

The tube 8c is made of resin that does not generate ions by heating and has low hygroscopicity. Thus, even in an environment with high temperature or humidity, the tube 8c does not generate gas. The tube 8c has a low amount of moisture absorption. This prevents water from coming into contact with the discharge member 8a. Thus, corrosion of the discharge member 8a can be prevented.

The tube 8c has flexibility. In this case, the tube 8c is not cut even if the discharge member 8a is twisted with respect to the terminal 8b. This reliably avoids cutting of the discharge member 8a at the end of the terminal 8b in assembling. If the tube 8c does not have flexibility, the discharge member 8a may be bent at the end of the tube 8c and cut in assembling, but such a possibility can be eliminated to facilitate assembling.

The tube is longer in the longitudinal direction of the discharge member 8a than the shortest distance between the terminal 8b and the end of the counter electrode 4. Thus, the tube 8c extends into the air trunk in which the discharge member 8a and the counter electrode 4 face each other. Specifically, the end of the tube 8c on the side of the discharge member 8a is placed closer to the air trunk than the end of the counter electrode 4. Thus, even if a voltage of 4 to 7 kV is applied between the discharge electrode 8 and the counter electrode 4, an abnormal discharge does not occur between the terminal 8b and the counter electrode 4.

The discharge member 8a and the terminal 8b are desirably made of metals having close potentials. A large potential difference between metals easily causes corrosion due to the potential difference. Thus, for example, when the discharge member 8a is made of tungsten, the terminal 8b is preferably made of tin, nickel, or tin-plated or nickel-plated metal. In this case, corrosion hardly occurs between dissimilar metals of the discharge member 8a and the terminal 8b. This increases durability of the discharge member 8a and the terminal 8b. The discharge member 8a may be tungsten having a carbon (graphite) layer. In this case, the discharge member 8a and the terminal 8b are not both metal, thereby preventing corrosion due to a potential difference. A desirable potential difference is about 0.1 V or less. When the terminal 8b is made of tin, SnO as an oxide is not dissolved in water and does not accelerate corrosion.

### Embodiment 1

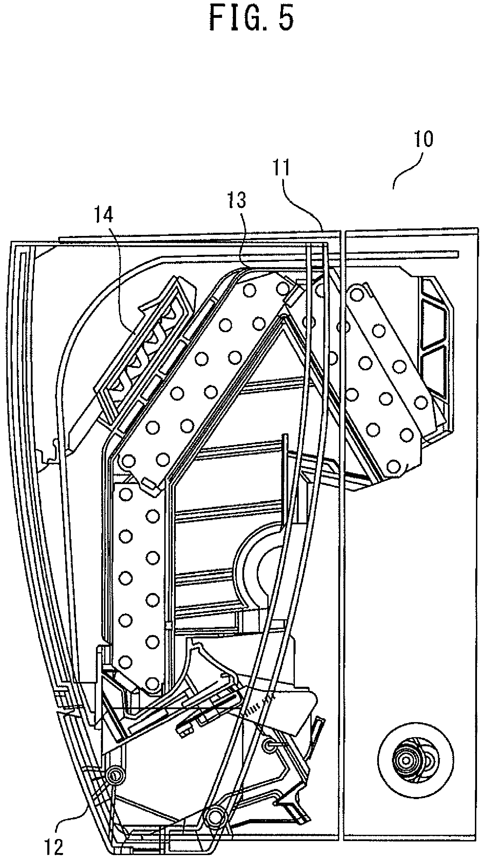

Figure 5 is a vertical sectional view of an indoor unit of an air conditioner including a discharge device according to Embodiment 1 of the present invention.

As shown in Figure 5, an inlet port 11 is formed in an upper surface of a body 10 of the inner unit of the air conditioner. An outlet port 12 is formed in a lower portion of a front surface of the body 10. In the body 10, a blast fan (not shown), a heat exchanger 13, and a discharge device 14 similar to the discharge device in Example 1 are provided.

For example, the blast fan is a propeller fan, a line flow fan, or the like. When the blast fan is a line flow fan, the heat exchanger 13 is placed above the outlet port 12 on a windward side of the blast fan. When the blast fan is a propeller fan, the heat exchanger 13 is placed above the outlet port 12 on a leeward side of the blast fan.

When the blast fan is a line flow fan, the discharge device 14 is placed on the windward side of the blast fan and a windward side of the heat exchanger 13. When the blast fan is a propeller fan, the discharge device 14 is placed on the leeward side of the blast fan and a windward side of the heat exchanger 13. The discharge device 14 is placed diagonally along a tilt of an upper surface of the heat exchanger 13. Thus, an opening surface of an upper frame 1 and an opening surface of a lower frame 2 in the discharge device 14 are tilted. Thus, a counter electrode 4 is placed almost vertically.

In the air conditioner, when the blast fan operates, air outside the body 10 is taken from the inlet port 11 into the body 10. The air taken into the body 10 is fed downward by the blast fan, and flows into the discharge device 14 or the heat exchanger 13.

The air fed from the blast fan diagonally flows into the discharge device 14, and moves in the discharge device 14 along the tilt of the counter electrode 4. The air discharged from the discharge device 14 flows into the heat exchanger 13. The air having passed through the heat exchanger 13 is discharged from the outlet port 12 to the outside of the body 10.

According to Embodiment 1 described above, the discharge device 14 placed in the body 10 can remove and inactivate bacteria, mold, viruses, or the like suspended in a room. The air fed from the blast fan diagonally flows into the discharge device 14. This prevents large pressure loss in the discharge device 14. Thus, even if the air conditioner includes the discharge device 14 therein, cooling and heating functions of the air conditioner are not significantly reduced.

### Industrial Applicability

The discharge device can be provided in products such as a room air conditioner, a package air conditioner, a cleaner, a hand drier, an air cleaner, a humidifier, a dehumidifier, or a refrigerator.

### Description of symbols

1 upper frame, 2 lower frame, 3 prefilter, 4 counter electrode, 5 first electrode support unit, 6 second electrode support unit, 7 power feeding unit, 8 discharge electrode, 8a discharge member, 8b terminal, 8c tube, 9 spring, 10 body, 11 inlet port, 12 outlet port, 13 heat exchanger, 14 discharge device

## Claims

1. An air conditioner comprising a discharge device comprising:
a discharge electrode which has a discharge member (8a); a loop-like terminal (8b) mounted to an end of the discharge member (8a) in a longitudinal direction; and a tube (8c) made of an electrically insulating resin that has flexibility and covering a mounting portion between an end of the discharge member (8a) and the terminal (8b), wherein an end of the tube (8c) is secured to the end of the discharge member (8a) and the terminal (8b) without any space therebetween by heat shrinkage or bonding, and
a counter electrode (4) facing the discharge member (8a), **characterized in that** the discharge member (8a) of the discharge electrode has a rectangular section and a potential difference between the discharge member (8a) and the terminal (8b) is 0.1 V or less.

2. The air conditioner according to claim 1, wherein the tube (8c) covers the discharge member (8a) from the mounting portion toward a center in the longitudinal direction of the discharge member (8a).

3. The air conditioner according to claim 1 or 2, wherein an end of a terminal (8b) on a side of the discharge member (8a) is placed outside the counter electrode (4) when seen in a facing direction of the discharge member (8a) and the counter electrode (4), and
the tube (8c) is longer in a longitudinal direction of the discharge member (8a) than a shortest distance (A) between the terminal (8b) and an end of the counter electrode (4).

4. The air conditioner according to any one of claims 1 to 3, further comprising:
a air trunk where through which air taken into the air conditioner passes, and wherein the tube (8c) extends into the air trunk in which the discharge member (8a) and the counter electrode (4) face each other.

5. The air conditioner according to claim 4, wherein an end of the tube (8c) on a side of the discharge member (8a) is placed closer to a center of the air trunk than the end of the counter electrode (4).

## Patentansprüche

1. Klimaanlage umfassend eine Entladungseinrichtung umfassend:
eine Entladungselektrode, die ein Entladungselement (8a) aufweist; einen schlaufenartigen Anschluss (8b), der in Längsrichtung an einem Ende des Entladungselements (8a) befestigt ist; und ein Rohr (8c) aus einem elektrisch isolierenden Harz, das Flexibilität aufweist und einen Befestigungsabschnitt zwischen einem Ende des Entladungselements (8a) und dem Anschluss (8b) abdeckt, wobei ein Ende des Rohrs (8c) am Ende des Entladungselements (8a) und des Anschlusses (8b) durch Wärmeschrumpfung oder -bindung ohne Zwischenraum fixiert ist,
und
eine Gegenelektrode (4), die dem Entladungselement (8a) zugewandt ist, **dadurch gekennzeichnet, dass** das Entladungselement (8a) der Entladungselektrode einen rechteckigen Abschnitt aufweist und eine Potentialdifferenz zwischen dem Entladungselement (8a) und dem Anschluss (8b) 0,1 V oder weniger beträgt.

2. Klimaanlage nach Anspruch 1, wobei das Rohr (8c) das Entladungselement (8a) vom Befestigungsabschnitt in Richtung einer Mitte in der Längsrichtung des Entladungselements (8a) abdeckt.

3. Klimaanlage nach Anspruch 1 oder 2, wobei ein Ende des Anschlusses (8b) auf einer Seite des Entladungselements (8a) bei Betrachtung in einer Blickrichtung des Entladungselements (8a) und der Gegenelektrode (4) außerhalb der Gegenelektrode (4) angeordnet ist, und
das Rohr (8c) in einer Längsrichtung des Entladungselements (8a) länger ist als der kürzeste Abstand (A) zwischen dem Anschluss (8b) und einem Ende der Gegenelektrode (4).

4. Klimaanlage nach einem der Ansprüche 1 bis 3, ferner umfassend:
einen Luftkanal, durch den die in die Klimaanlage aufgenommene Luft strömt, und wobei sich das Rohr (8c) in den Luftkanal erstreckt, in dem das Entladungselement (8a) und die Gegenelektrode (4) einander gegenüberliegen.

5. Klimaanlage nach Anspruch 4, wobei ein Ende des Rohrs (8c) auf einer Seite des Entladungselements (8a) näher an einer Mitte des Luftkanals angeordnet ist als das Ende der Gegenelektrode (4).

## Revendications

1. Climatiseur comprenant un dispositif de décharge comprenant :
une électrode de décharge qui a un élément de décharge (8a) ; une borne en forme de boucle (8b) montée sur une extrémité de l'élément de décharge (8a) dans une direction longitudinale ; et un tube (8c) réalisé avec une résine électriquement isolante qui présente une flexibilité et recouvrant une partie de montage entre une extrémité de l'élément de décharge (8a) et la borne (8b), dans lequel une extrémité du tube (8c) est fixée à l'extrémité de l'élément de décharge (8a) et la borne (8b) sans aucun espace entre elles par thermorétraction ou collage, et
une électrode auxiliaire (4) faisant face à l'élément de décharge (8a), **caractérisé en ce que** l'élément de décharge (8a) de l'électrode de décharge a une section rectangulaire et une différence potentielle entre l'élément de décharge (8a) et la borne (8b) est de 0,1 V ou moins.

2. Climatiseur selon la revendication 1, dans lequel le tube (8c) recouvre l'élément de décharge (8a) à partir de la partie de montage vers un centre dans la direction longitudinale de l'élément de décharge (8a).

3. Climatiseur selon la revendication 1 ou 2, dans lequel une extrémité d'une borne (8b) sur un côté de l'élément de décharge (8a) est placée à l'extérieur de l'électrode auxiliaire (4) lorsqu'elle est observée dans une direction de face de l'élément de décharge (8a) et de l'électrode auxiliaire (4), et
le tube (8c) est plus long dans une direction longitudinale de l'élément de décharge (8a) que la plus courte distance (A) entre la borne (8b) et une extrémité de l'électrode auxiliaire (4).

4. Climatiseur selon l'une quelconque des revendications 1 à 3, comprenant en outre :
une manchette à travers laquelle passe l'air prélevé dans le climatiseur, et
dans lequel le tube (8c) s'étend dans la manchette dans laquelle l'élément de décharge (8a) et l'électrode auxiliaire (4) se font face.

5. Climatiseur selon la revendication 4, dans lequel une extrémité du tube (8c) sur un côté de l'élément de décharge (8a) est placée plus à proximité d'un centre de la manchette que l'extrémité de l'électrode auxiliaire (4).
